# EUROPEAN PATENT APPLICATION

(11) **EP 4 782 830 A1**
(43) Date of publication of application: **29.07.2026**
(21) Application number: 26152808.7
(22) Date of filing: 20.01.2026
(51) Int. Cl.: G01N 33/00

(54) **CALIBRATION SYSTEM AND METHOD FOR A GAS DETECTOR**

(30) Priority: 27.01.2025 CN 202510126463
(71) Applicant: Life Safety Distribution GmbH, 1180 Rolle (CH)
(72) Inventor: HUANG, Chuang, Charlotte, 28202 (US); XU, Yuhui, Charlotte, 28202 (US); HU, Yu, Charlotte, 28202 (US); HUANG, Liuxin, Charlotte, 28202 (US)
(74) Representative: Haseltine Lake Kempner LLP

(57) **Abstract**

A calibration system and a method for a gas detector are provided. The calibration system includes a diffusion chamber having a first side coupled to the gas detector and a second side coupled to a filter. A container is coupled to the diffusion chamber via a valve and includes an absorbent retaining a gas and a heating element to heat the absorbent to release the gas from the absorbent to the diffusion chamber through the valve. At least one processor is configured to regulate the valve to release the gas, determine a change in a signal reading of the gas detector associated with a diffusion of gas from the filter, determine a sensitivity drift factor based on the change in the signal reading, determine a change in concentration of the gas based on the sensitivity drift factor, and calibrate the gas detector based on the change in concentration.

## Description

### TECHNOLOGICAL FIELD

Example embodiments of the present disclosure generally relates to a calibration system, and more particularly relates to a calibration system for a gas detector.

### BACKGROUND

Accurate performance of gas detectors is crucial for ensuring reliable measurements in various applications. To maintain accuracy, it is essential for the gas detectors to undergo calibration at regular intervals. However, the process of calibration presents several challenges, particularly for fixed gas detectors compared to portable gas detectors. Fixed gas detectors are often stationary, hence impractical to transport to a calibration laboratory. Further, the fixed positioning of such fixed gas detectors hinders operational efficiency during calibration and pose safety risks, especially in hazardous environments.

The inventors have identified numerous areas of improvement in the existing technologies and processes, which are the subjects of embodiments described herein. Through applied effort, ingenuity, and innovation, many of these deficiencies, challenges, and problems have been solved by developing solutions that are included in embodiments of the present disclosure, some examples of which are described in detail herein.

### BRIEF SUMMARY

The following presents a simplified summary in order to provide a basic understanding of some aspects of the present disclosure. This summary is not an extensive overview and is intended to neither identify key or critical elements nor delineate the scope of such elements. Its purpose is to present some concepts of the described features in a simplified form as a prelude to the more detailed description that is presented later.

In an example embodiment, a calibration system for a gas detector is disclosed. The calibration system comprises a diffusion chamber having a first side coupled to the gas detector and a second side coupled to a filter, a container coupled to the diffusion chamber via a valve. Further, the container includes an absorbent configured to retain a gas and a heating element configured to heat the absorbent to release the gas from the absorbent to the diffusion chamber through the valve. The calibration system further comprises at least one processor communicatively coupled to the valve and the heating element. Further, the at least one processor is configured to regulate the valve to release the gas into the diffusion chamber, determine a change in a signal reading of the gas detector associated with a diffusion of gas from the filter, determine a sensitivity drift factor of the gas detector based at least on the change in the signal reading of the gas detector, determine a change in concentration of the gas based at least on the sensitivity drift factor for calibrating the gas detector, and calibrate the gas detector based at least on the change in concentration.

In some embodiments, the at least one processor is further configured to regulate the valve for a predefined time interval to release the gas into the diffusion chamber based on activation of the heating element.

In some embodiments, the change in the signal reading of the gas detector corresponds to a difference between a final signal reading and an initial signal reading. In some embodiments, the final signal reading corresponds to a signal reading of the gas detector after a predefined time interval and the initial signal reading corresponds to the signal reading of the gas detector during the predefined time interval.

In some embodiments, the at least one processor is further configured to determine the sensitivity drift factor of the gas detector based at least on the initial signal reading, the final signal reading, and an index. In some embodiments, the sensitivity drift factor corresponds to an amount by which sensitivity of measurement of the gas detector varies as ambient conditions change. In some embodiments, the change in concentration of the gas is a product of a current signal reading of the gas detector and an updated index. Further, the updated index is the index plus the sensitivity drift factor.

In some embodiments, the filter is configured to reduce the concentration of the gas within the diffusion chamber by diffusing the gas from the diffusion chamber. In some embodiments, the valve corresponds but not limited to an electromagnetic valve.

In some embodiments, the filter is made of at least one of a ventilate film like ePTEF (Expanded polytetrafluoroethylene) film and TPU (thermoplastic polyurethanes) film. In some embodiments, the absorbent is made of at least one of a material like activated carbon, metal organic frameworks, zeolites, activated alumina, molecular sieve, silica gel etc.

In another example embodiment, a method is disclosed. The method comprising steps of regulating, via at least one processor communicatively coupled to a valve and a heating element, the valve to release a gas into a diffusion chamber. Further, the diffusion chamber has a first side coupled to the gas detector and a second side coupled to a filter. Further, a container is coupled to the diffusion chamber via the valve. Further, the container includes an absorbent configured to retain the gas and a heating element configured to heat the absorbent to release the gas from the absorbent to the diffusion chamber through the valve; determining, via the at least one processor, a change in a signal reading of the gas detector associated with a diffusion of gas from the filter; determining, via the at least one processor, a sensitivity drift factor of the gas detector based at least on the change in the signal reading of the gas detector; determining, via the at least one processor, a change in concentration of the gas based at least on the sensitivity drift factor, for calibrating the gas detector; and calibrating, via the at least one processor, the gas detector based at least on the change in concentration.

The above summary is provided merely for purposes of summarizing some example embodiments to provide a basic understanding of some aspects of the present disclosure. Accordingly, it will be appreciated that the above-described embodiments are merely examples and should not be construed to narrow the scope or spirit of the present disclosure in any way. It will be appreciated that the scope of the present disclosure encompasses many potential embodiments in addition to those here summarized, some of which will be further described below.

### BRIEF DESCRIPTION OF THE DRAWINGS

Having thus described certain example embodiments of the present disclosure in general terms, reference will hereinafter be made to the accompanying drawings, which are not necessarily drawn to scale, and wherein:
FIG. 1 illustrates a schematic diagram of a calibration system for a gas detector in accordance with an example embodiment of the present disclosure;
FIG. 2 illustrates a graph representing change in a concentration of gas determined by the gas detector in accordance with an example embodiment of the present disclosure; and
FIG. 3 illustrates a flowchart showing a method of calibrating the gas detector in accordance with an example embodiment of the present disclosure.

### DETAILED DESCRIPTION

Some embodiments will now be described more fully hereinafter with reference to the accompanying drawings, in which some, but not all, embodiments are shown. Indeed, various embodiments may be embodied in many different forms and should not be construed as limited to the embodiments set forth herein; rather, these embodiments are provided so that this disclosure will satisfy applicable legal requirements.

The components illustrated in the figures represent components that may or may not be present in various embodiments of the present disclosure described herein such that embodiments may include fewer or more components than those shown in the figures while not departing from the scope of the present disclosure. Some components may be omitted from one or more figures or shown in dashed line for visibility of the underlying components.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in various embodiments," "in one embodiment," "according to one embodiment," "in some embodiments," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments or it may be excluded.

The present disclosure provides various embodiments of a calibration system for a gas detector. Embodiments of the present disclosure may comprise a diffusion chamber having a first side may be coupled to the gas detector and a second side may be coupled to a filter. Embodiments of the present disclosure may comprise a container coupled to the diffusion chamber via a valve. The container may include an absorbent configured to retain a gas and a heating element configured to heat the absorbent to release the gas from the absorbent to the diffusion chamber through the valve. Embodiments of the present disclosure may comprise at least one processor communicatively coupled to the valve and the heating element. The at least one processor may be configured to regulate the valve to release the gas into the diffusion chamber, determine a change in a signal reading of the gas detector associated with a diffusion of gas from the filter, determine a sensitivity drift factor of the gas detector based at least on the change in the signal reading of the gas detector, determine a change in concentration of the gas based at least on the sensitivity drift factor for calibrating the gas detector, and calibrate the gas detector based at least on the change in concentration.

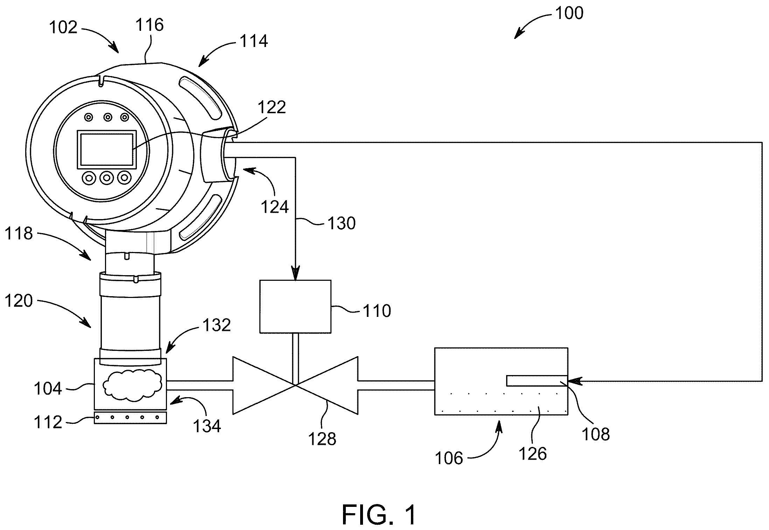

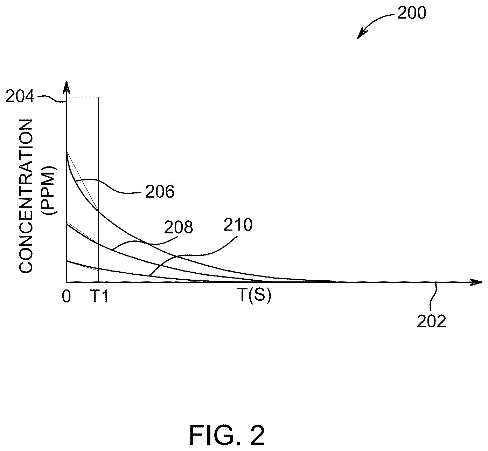

FIG. 1 illustrates a schematic diagram of a calibration system 100 for a gas detector 102, in accordance with an example embodiment of the present disclosure. FIG. 2 illustrates a graph 200 representing change in a concentration of a gas determined by the gas detector 102, in accordance with an example embodiment of the present disclosure.

In some embodiments, the calibration system 100 may comprise a diffusion chamber 104, a container 106, a heating element 108, and at least one processor 110. In some embodiments, the diffusion chamber 104 may be coupled with a filter 112. In some embodiments, the gas detector 102 may be installed at a specific location. In some embodiments, the specific location may comprise industrial plants and factories, manufacturing facilities, labs and research centers, mining operations, waste water treatment plants, commercial buildings, warehouses etc. In some embodiments, the gas detector 102 may correspond to a fix gas detector. In some embodiments, the fix gas detector may be permanently installed in a fix position at the specific location to monitor presence of hazardous gases or gas leakage, emission in proximate to the specific location.

As illustrated in FIG. 1, the gas detector 102 may comprise an enclosure 114, a mounting bracket 116, a gas sampling inlet 118. Further, the gas detector 102 may comprise at least one sensor 120, an electronic module (not shown), and a display screen 122. In some embodiments, the enclosure 114 may be configured to house one or more components (e.g., the at least one sensor 120, electronic module, display screen 122 etc.). In some embodiments, the enclosure 114 of the gas detector 102 may be configured to prevent damage to the one or more components from various hazards. Further, the hazards may include but not limited to extreme temperature, humidity, mechanical stress, vibrations etc. In some embodiments, the mounting bracket 116 of the gas detector 102 may be configured to allow mounting of the gas detector 102 at a fixed surface (e.g., wall, ceiling, panels, doors etc.).

In some embodiments, the gas sampling inlet 118 of the gas detector 102 may be configured to receive a target gas present in proximity to the gas detector 102. Further, the gas sampling inlet 118 may be integrated with the at least one sensor 120. Further, the at least one sensor 120 may be configured to interact with the target gas present in proximate to the specific location, resulting into change in electrical properties like voltage or current. The change in electrical properties may be measured by the electronic module of the gas detector 102. The electronic module may comprise one or more electronic components. Further, the electronic module may be configured to translate the change in electrical properties into a gas concentration value or reading. The electronics module may process signals from the at least one sensor 120 and convert the signals into the gas concentration value or reading. In some embodiments, the display screen 122 of the gas detector 102 may be configured to display the gas concentration value or reading.

In some embodiments, the gas detector 102 may further comprise a communication port 124. Further, the communication port 124 of the gas detector 102 may be configured to allow connection of the gas detector 102 with various external systems (not shown). In one example, the gas detector 102 may be connected with a power supply (not shown). Further, the gas detector 102 may be hardwired to the power supply for power backup. In some instances, to maintain accuracy, the gas detector 102 may undergo calibration at regular intervals. Further, the calibration system 100 may be configured to perform calibration of the gas detector 102.

As illustrated in FIG. 1, the calibration system 100 may comprise the diffusion chamber 104. In some embodiments, the diffusion chamber 104 may be coupled with at least one side of the gas detector 102. In an example, the diffusion chamber 104 may be installed at a bottom side of the gas detector 102. In an example, the diffusion chamber 104 may be coupled to the gas sampling inlet 118 of the gas detector 102. In some embodiments, the diffusion chamber 104 may serve as a controlled space where the target gas and/or other gases interacts with the gas detector 102. In one example, the diffusion chamber 104 corresponds to the controlled space that may be isolated from external environmental factors for measurement and calibration purposes.

In some embodiments, the diffusion chamber 104 may be constructed with various materials. The materials for constructing the diffusion chamber 104 may ensure that an internal surface of the diffusion chamber 104 remain isolated from the external environment. In some embodiments, the diffusion chamber 104 may comprise a first side 132 and a second side 134. In one example, the first side 132 of the diffusion chamber 104 may correspond to a top side of the diffusion chamber 104. In some embodiments, the first side 132 of the diffusion chamber 104 may be coupled to the gas detector 102. In another example, the second side 134 of the diffusion chamber 104 may correspond to a bottom side of the diffusion chamber 104. In some embodiments, the second side 134 of the diffusion chamber 104 may be coupled to the filter 112. In some embodiments, the filter 112 may be configured to enable diffusion of gases from the diffusion chamber 104 to an external environment.

In some embodiments, the calibration system 100 may comprise the container 106. In some embodiments, the container 106 may be coupled with the diffusion chamber 104. In some embodiments, the container 106 may include an absorbent 126. In some embodiments, the absorbent 126 may be configured to retain a gas. The absorbent 126 may be composed of various materials that are capable of retaining and gradually releasing the gas. In some embodiments, the absorbent 126 is made of at least one of a material (e.g., activated carbon, metal organic frameworks, zeolites, activated alumina, molecular sieve, silica gel etc.). In an example, the gas may comprise, but is not limited to, methane, isobutene, NO2 or other gases used in calibration.

In some embodiments, the container 106 may be coupled to the diffusion chamber 104 through a valve 128. In some embodiments, the valve 128 may comprise but not limited to an electromagnetic valve, a solenoid valve etc. In some embodiments, the valve 128 may be electronically controlled to regulate flow of the gas released from the absorbent 126. Further, the released gas may travel from the container 106 to the diffusion chamber 104 through the valve 128. In an example, the valve 128 may comprise a solenoid and a plunger. The solenoid may move the plunger to open or close the valve 128. In an example, when current flows through the solenoid, a magnetic field is generated that moves the plunger to open or close the valve 128.

In some embodiments, the calibration system 100 may comprise the at least one processor 110. In some embodiments, the at least one processor 110 may be configured to control a self-calibration process of the gas detector 102. The at least one processor 110 may include suitable logic, input/ output circuitry, and communication circuitry that are operable to execute one or more instructions stored in a memory to perform predetermined operations. The at least one processor 110 may be configured to execute one or more computer-readable program instructions, such as program instructions to carry out any of the functions described in this description. Further, the at least one processor 110 may be implemented using one or more technologies known in the art. Examples of the at least one processor 110 include, but are not limited to, one or more general purpose processors and/or one or more special purpose processors.

In some embodiments, the heating element 108 may be installed within the container 106. Further, the heating element 108 may be communicatively coupled with the at least one processor 110. In an example, the heating element 108 may be positioned in contact with the absorbent 126 inside the container 106. In an example, the heating element 108 may correspond to a resistance wire heater, cartridge heater, infrared heater, induction heater etc. In some embodiments, the at least one processor 110 may be configured to activate the heating element 108. Upon activating the heating element 108, temperature of the absorbent 126 increases as the heating element 108 is positioned in contact with the absorbent 126. The increase in temperature of the absorbent 126 causes increase in kinetic energy of molecules of the gas retained in the absorbent 126. Further, due to increase in the kinetic energy, intermolecular forces between the molecules of the gas weaken, causing the gas to desorb or release from absorbent 126. Upon being released form the absorbent 126, the gas may flow towards the diffusion chamber 104. In some embodiments, the absorbent 126 is made of at least one of a material (e.g., activated carbon, metal organic frameworks, zeolites, activated alumina, molecular sieve, silica gel etc.)

In some embodiments, the heating element 108 may be powered by the gas detector 102 through a power cable 130. In some embodiments, the valve 128 may be powered by the gas detector 102 via the power cable 130. In an example, the heating element 108 and the valve 128 may be electrically powered by the gas detector 102 through the power cable 130, respectively, drawing energy from one or more internal power source of the gas detector 102. In an example, the one or more internal power source of the gas detector 102 may correspond to a rechargeable battery, capacitors or a lithium ion battery.

In some embodiments, the valve 128 may be communicatively coupled with the at least one processor 110. Further, upon activating the heating element 108, the at least one processor 110 subsequently regulates the valve 128 for a predefined time interval to release a concentration of the gas from the container 106 into the diffusion chamber 104. The at least one processor 110 is further configured to regulate the valve 128 for a predefined time interval to release the gas into the diffusion chamber 104 based on activation of the heating element 108.

In some embodiments, the at least one processor 110 may be configured to activate the gas detector 102 for a predefined time interval, upon releasing of the gas into the diffusion chamber 104. Further, the gas detector 102 may be configured to determine an initial reading of the concentration of the gas released from the container 106 into the diffusion chamber 104. In one example, the at least one processor 110 may be configured to deactivate the gas detector 102 upon receiving the initial reading of the concentration of the gas.

In some embodiments, the filter 112 may be coupled to the second side 134 of the diffusion chamber 104. In an example, the filter 112 may be coupled to the bottom side of the diffusion chamber 104. In some embodiments, when the gas reaches the diffusion chamber 104, the filter 112 may allow diffusion of the gas out of the diffusion chamber 104. In some embodiments, the filter 112 may be configured to enable movement of the target gas between the diffusion chamber 104 and the external environment. In one example, the filter 112 may comprise a plurality of pores that may enable diffusion of the target gas from the diffusion chamber 104. In some embodiments, the plurality of pores may be configured to allow molecules of the target gas to pass through while potentially filtering out larger particles.

In some embodiments, the filter is made of at least one of a ventilate film (e.g., ePTEF (Expanded polytetrafluoroethylene) film and TPU (thermoplastic polyurethanes) film). In some embodiments, the absorbent is made of at least one of a material (e.g., activated carbon, metal organic frameworks, zeolites, activated alumina, molecular sieve, silica gel etc.). Further, due to diffusion of the gas through the filter 112, the concentration of the gas inside the diffusion chamber 104 may change. In some embodiments, the filter 112 may be configured to reduce the concentration of the gas within the diffusion chamber 104 by diffusing the gas from the diffusion chamber 104. In some embodiments, the diffusion of the gas through the filter 112, out of the diffusion chamber 104, may provide a controlled environment inside the diffusion chamber 104 to measure different concentration of the gas for calibration.

In some embodiments, after diffusion of the gas from the diffusion chamber 104 to the external environment, the at least one processor 110 may be configured to activate the gas detector 102. Further, the at least one processor 110 may be configured to determine a final reading of the gas present inside the diffusion chamber 104 after diffusion of the gas through the filter. Further, the at least one processor 110 may be configured to measure change in concentration of the gas inside the diffusion chamber 104. In some embodiments, the at least one processor 110 may activate the gas detector 102 to determine a change in a signal reading of the gas detector 102 associated with a diffusion of the gas from the diffusion chamber 104 through the filter 112. In some embodiments, the change in the signal reading of the gas detector 102 may correspond to a difference between the final signal reading and the initial signal reading. In some embodiments, the final reading may correspond to a signal reading of the gas detector 102 after the predefined time interval. Further, the initial signal reading may correspond to the signal reading of the gas detector 102 during the predefined time interval.

As illustrated in FIG. 2, the graph 200 represents change in concentration of one or more gases inside the diffusion chamber 104. Further, each gas filled in the diffusion chamber 104 may comprise a different concentration level. In some embodiments, the graph 200 may comprise an X-axis 202 representing diffusion time (0-T(S)) and a Y-axis 204 representing different concentration value. Further, the graph 200 may comprise one or more trends. Further, each of the one or more trends may be associated with the one or more gases. In some embodiments, a trend 206 may be associated with a first gas, a trend 208 may be associated with a second gas, and a trend 210 may be associated with a third gas. Further, the diffusion time may comprise a first time interval (0-T1). Further, the first time interval may represent a concentration value of the one or more gases, when the one or more gases are released into the diffusion chamber 104.

Further, the filter 112 coupled to the diffusion chamber 104 may be configured to enable diffusion of the one or more gases from the diffusion chamber 104 to the external environment. In some embodiments, upon diffusion of the one or more gases, the concentration value of each of the one or more gases reduces. Further, the diffusion time may comprise a second time interval (T(S)-T1). As illustrated in the graph 200, the concentration value of the one or more gases reduces during the second time interval. In some embodiments, the graph 200 may state that the change in concentration of the gas is directly proportional to derivative of difference in concentration of the gas between the first time interval and the second time interval.

In some embodiments, the at least one processor 110 may determine the sensitivity drift factor of the gas detector 102 based at least on the change in the signal reading of the gas detector 102. In one example, the at least one processor 110 is configured to determine the sensitivity drift factor of the gas detector 102 based at least on the initial signal reading, the final reading, and an index. In some embodiments, the sensitivity drift factor may correspond to an amount by which sensitivity of measurement of the gas detector 102 varies as ambient conditions change. In various examples, the ambient conditions may comprise change in temperature, change in humidity etc. In some embodiments, the at least one processor 110 may determine the sensitivity drift factor of the gas detector 102 based at least on the index (i.e. base sensitivity index) of the gas detector 102, the initial signal reading associated with the gas concentration inside the diffusion chamber 104 at the predefined time interval and the final signal reading associated with the gas concentration inside the diffusion chamber 104 after the predefined time interval.

In some embodiments, the at least one processor 110 may be configured to determine an updated index upon determining the sensitivity drift factor. Further, the updated index may correspond to a sum of the index and the sensitivity drift factor. In some embodiments, the at least one processor 110 may determine change in the concentration of the gas based at least on the sensitivity drift factor for calibrating the gas detector 102. In some embodiments, the change in concentration of the gas may be a product of a current signal reading of the gas detector 102 and the updated index. In one example, the gas detector 102 may detect the change in concentration of the gas and display a gas concentration measurement value on the display screen 122. In some embodiments, the at least one processor 110 may be configured to calibrate the gas detector 102 based at least on the change in concentration.

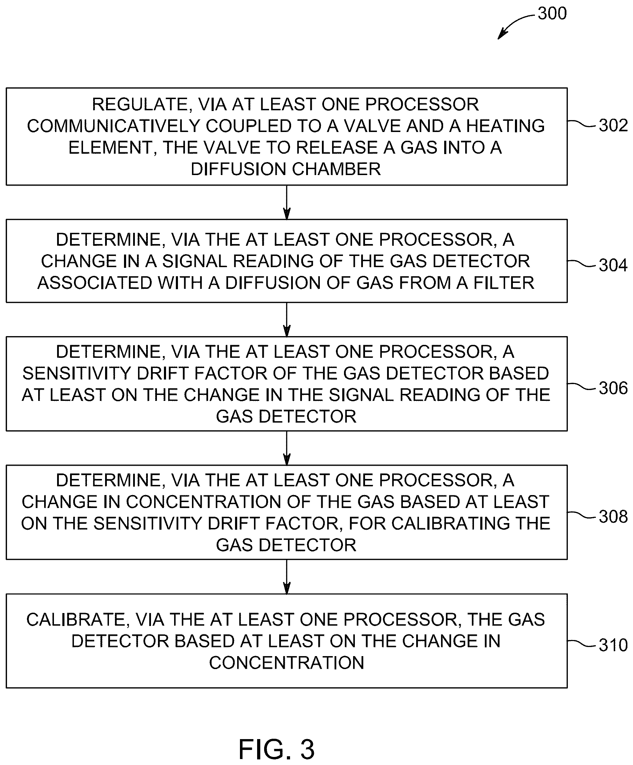

FIG. 3 illustrates a flowchart 300 showing a method of calibrating the gas detector 102, in accordance with an example embodiment of the present disclosure.

At operation 302, the at least one processor 110 may be configured to regulate the valve 128 to release the gas into the diffusion chamber 104. Further, the diffusion chamber 104 may have the first side 132 coupled to the gas detector 102 and the second side 134 coupled to the filter 112. In some embodiments, the container 106 may be coupled to the diffusion chamber 104 via the valve 128. Further, the container 106 may include the absorbent 126. Further, the absorbent 126 may be configured to retain the gas. In some embodiments, the at least one processor 110 may be configured to regulate the valve 128 for the predefined time interval to release the gas into the diffusion chamber 104 based on activation of the heating element 108. Further, the heating element 108 is configured to heat the absorbent 126 to release the gas from the container 106 to the diffusion chamber 104 through the valve 128. In some embodiments, the valve 128 may comprise an electromagnetic valve, solenoid valve etc. In one example, a change in pressure between the container 106 and the diffusion chamber 104 may facilitate transferring of the gas from the container 106 to the diffusion chamber 104.

Further, upon releasing of the gas from the container 106 to the diffusion chamber 104, the at least one processor 110 may be configured to activate the gas detector 102. Further, the gas detector 102 may be configured to determine the initial signal (i.e. the gas concentration measurement value) reading at the predefined time interval. Further, the diffusion chamber 104 may be coupled with the filter 112. Further, the filter 112 may be configured to enable diffusion of the gas released through the valve 128. Further, the concentration of the gas may reduce after diffusion through the filter. Further, upon diffusion of the gas from the container 106 through the filter 112, the at least one processor 110 may again activate the gas detector 102. Further, the gas detector 102 may be configured to determine the final signal reading (i.e. the gas concentration measurement value) after the predefined time interval.

At operation 304, the at least one processor 110 may be configured to determine the change in the signal reading of the gas detector 102 associated with the diffusion of gas from the filter 112. In some embodiments, the change in the signal reading of the gas detector 102 may correspond to the difference between the final signal reading and the initial signal reading. Further, the final signal reading may correspond to the signal reading of the gas detector 102 after the predefined time interval and the initial signal reading may correspond to the signal reading of the gas detector 102 during the predefined time interval.

At operation 306, the at least one processor 110 may be configured to determine the sensitivity drift factor of the gas detector 102 based at least on the change in the signal reading of the gas detector 102. In some embodiments, the at least one processor 110 may be configured to use a gas detector algorithm to determine the sensitivity drift factor of the gas detector 102. In some embodiments, the at least one processor 110 may determine the sensitivity drift factor of the gas detector 102 based at least on the initial signal reading, the final signal reading, and the index. Further, the sensitivity drift factor may correspond to the amount by which sensitivity of measurement of the gas detector 102 varies as the ambient conditions change. Further, the at least one processor 110 may be configured to evaluate the updated index upon determining the sensitivity drift factor. Further, the updated index may correspond to a sum of the index and the sensitivity drift factor.

At operation 308, the at least one processor 110 may be configured to determine the change in concentration of the gas based at least on the sensitivity drift factor, for calibrating the gas detector 102. In some embodiments, the change in concentration of the gas may be a product of a current signal reading of the gas detector 102 and the updated index. Further, the updated index is the index plus the sensitivity drift factor. Further, the at least one processor 110 may determine an updated index of the gas based at least on the sensitivity drift factor determined. In some embodiments, the at least one processor 110 may determine the updated index of the gas detector 102 based at least on the sensitivity drift factor of the gas detector 102 determined and the base sensitivity index of the gas detector 102.

At operation 310, the at least one processor 110 may be configured to calibrate the gas detector 102 based at least on the change in concentration. In some embodiments, the at least one processor 110 may be configured to calibrate the gas detector 102 using the updated index. In one example, the gas detector 102 may be configured to provide an accurate concentration of gas after successful calibration.

Embodiments may be configured to calibrate the gas detector 102 in case of change in the measurement sensitivity of the gas detector 102. The embodiments may be configured to determine the sensitivity drift factor and the updated calibration index of the gas detector 102. The embodiments may be configured to update the base sensitivity index in the gas detector 102 algorithm with the updated calibration index to account for change in the measurement sensitivity of the gas detector 102.

Many modifications and other embodiments of the present disclosure set forth herein will come to mind to one skilled in the art to which this present disclosure pertains having the benefit of the teachings presented in the foregoing descriptions and the associated drawings. Therefore, it is to be understood that the present disclosure is not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A calibration system for a gas detector, the calibration system comprising:
a diffusion chamber having a first side coupled to the gas detector and a second side coupled to a filter;
a container coupled to the diffusion chamber via a valve, wherein the container includes an absorbent configured to retain a gas and a heating element configured to heat the absorbent to release the gas from the absorbent to the diffusion chamber through the valve; and
at least one processor communicatively coupled to the valve and the heating element, wherein the at least one processor is configured to:
regulate the valve to release the gas into the diffusion chamber; and
determine a change in a signal reading of the gas detector associated with a diffusion of gas from the filter;
determine a sensitivity drift factor of the gas detector based at least on the change in the signal reading of the gas detector;
determine a change in concentration of the gas based at least on the sensitivity drift factor for calibrating the gas detector; and
calibrate the gas detector based at least on the change in concentration.

2. The calibration system of claim 1, wherein the at least one processor is further configured to regulate the valve for a predefined time interval to release the gas into the diffusion chamber based on activation of the heating element.

3. The calibration system of claim 1, wherein the change in the signal reading of the gas detector corresponds to a difference between a final signal reading and an initial signal reading.

4. The calibration system of claim 3, wherein the final signal reading corresponds to a signal reading of the gas detector after a predefined time interval and the initial signal reading corresponds to the signal reading of the gas detector during the predefined time interval.

5. The calibration system of claim 3, wherein the at least one processor is further configured to determine the sensitivity drift factor of the gas detector based at least on the initial signal reading, the final signal reading, and an index.

6. The calibration system of claim 5, wherein the sensitivity drift factor corresponds to an amount by which sensitivity of measurement of the gas detector varies as ambient conditions change.

7. The calibration system of claim 5, wherein the change in concentration of the gas is a product of a current signal reading of the gas detector and an updated index, wherein the updated index is the index plus the sensitivity drift factor.

8. The calibration system of claim 1, wherein the filter is configured to reduce the concentration of the gas within the diffusion chamber by diffusing the gas from the diffusion chamber.

9. A method for calibrating a gas detector, the method comprising:
regulating, via at least one processor communicatively coupled to a valve and a heating element, the valve to release a gas into a diffusion chamber, wherein the diffusion chamber has a first side coupled to the gas detector and a second side coupled to a filter, wherein a container is coupled to the diffusion chamber via the valve, wherein the container includes an absorbent configured to retain the gas and a heating element configured to heat the absorbent to release the gas from the absorbent to the diffusion chamber through the valve;
determining, via the at least one processor, a change in a signal reading of the gas detector associated with a diffusion of gas from the filter;
determining, via the at least one processor, a sensitivity drift factor of the gas detector based at least on the change in the signal reading of the gas detector;
determining, via the at least one processor, a change in concentration of the gas based at least on the sensitivity drift factor for calibrating the gas detector; and
calibrating, via the at least one processor, the gas detector based at least on the change in concentration.

10. The method of claim 9 further comprising regulating, via the at least one processor, the valve for a predefined time interval to release the gas into the diffusion chamber based on activation of the heating element.

11. The method of claim 9, wherein the change in the signal reading of the gas detector corresponds to a difference between a final signal reading and an initial signal reading.

12. The method of claim 11, wherein the final signal reading corresponds to signal reading of the gas detector after a predefined time interval and the initial signal reading corresponds to the signal reading of the gas detector during the predefined time interval.

13. The method of claim 11, further comprising, determining, via the at least one processor, the sensitivity drift factor of the gas detector is based at least on the initial signal reading, the final signal reading, and an index.

14. The method of claim 13, wherein the sensitivity drift factor corresponds to an amount by which sensitivity of measurement of the gas detector varies as ambient conditions change.

15. The method of claim 13, wherein the change in concentration of the gas is a product of a current signal reading of the gas detector and an updated index, wherein the updated index is the index plus the sensitivity drift factor.
